Europäisches Patentamt

European Patent Office

Office européen des brevets

(11) Veröffentlichungsnummer: **0 508 345 A2**

# EUROPÄISCHE PATENTANMELDUNG

(21) Anmeldenummer: **92105916.8**

(22) Anmeldetag: **06.04.92**

(51) Int. Cl.5: **C07C 49/92**, C01F 11/00

(30) Priorität: **09.04.91 DE 4111460**

(43) Veröffentlichungstag der Anmeldung:
**14.10.92 Patentblatt 92/42**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Becker, Winfried, Dr.**
**Am Eschenhang 7**
**W-6239 Bremthal(DE)**
Erfinder: **Weidlich, Stephan**
**Dürkheimer Strasse 37**
**W-6230 Frankfurt am Main 80(DE)**

(54) **Flüchtiger Erdalkali - Komplex und seine Verwendung.**

(57) Es wird ein Erdalkali-Chelatkomplex des 2,2,6,6-Tetramethylheptandions beschrieben mit der allgemeinen Formel (I)

$$M^{2+}(C_{11}H_{19}O_2)^-_2 \cdot L_k \qquad (I)$$

wobei M für Calcium, Strontium oder Barium L für einen Liganden und k für eine Zahl von 1 bis 3 steht, der frei ist von Kristallwasser und bei dem der Ligand L ein aliphatischer Ether mit mindestens zwei Sauerstoffatomen im Molekül ist. Es wird auch ein Verfahren zur Herstellung des Chelatkomplexes beschrieben, sowie seine Verwendung bei der Herstellung von beschichteten Substraten.

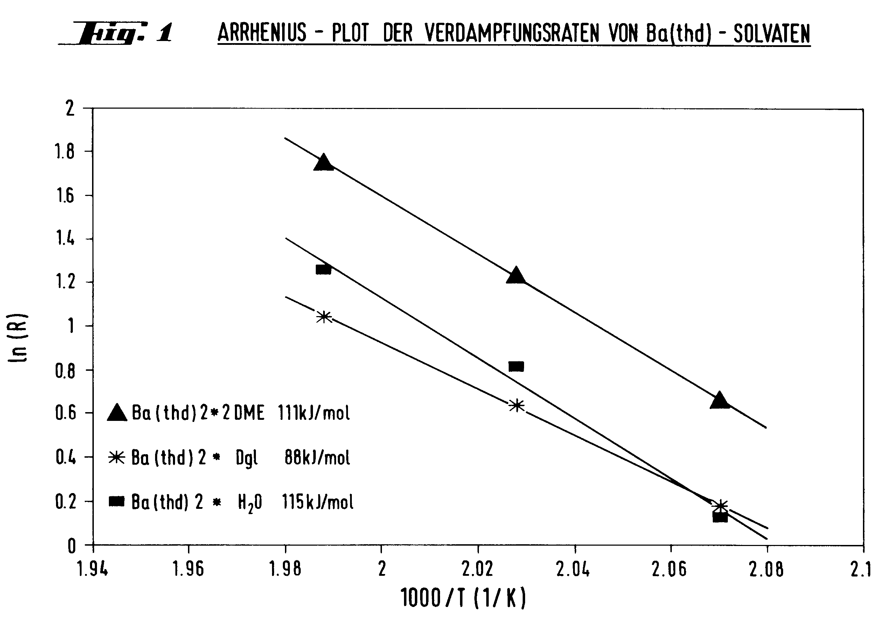

Fig. 1   ARRHENIUS – PLOT DER VERDAMPFUNGSRATEN VON Ba(thd) – SOLVATEN

EP 0 508 345 A2

Die Erfindung betrifft einen Erdalkali-Chelatkomplex des 2,2,6,6-Tetramethylheptandions, der aliphatische Äther als Komplexliganden enthält und daher eine besonders hohe Flüchtigkeit aufweist. Die Erfindung betrifft ferner die Verwendung dieses Chelatkomplexes zur Beschichtung von Substraten mit Erdalkali enthaltendem Hochtemperatur-Supraleiter-Material.

Seit der Entdeckung der supraleitenden Eigenschaften des Lanthanbariumcuprats durch Bednorz und Müller sind eine Vielzahl weiter supraleitender Oxidkeramiken entdeckt worden, deren kritische Temperaturen z. T. oberhalb der Siedetemperatur des Stickstoffs liegen. Sie sind daher von großem technischen Interesse.

Die neuen Supraleiter aus Oxid-Keramik besitzen granulare Struktur. Die Korngrenzen stellen für den supraleitenden Strom sogenannte "weak-links" dar, die die kritischen Stromdichten in polykristalliner Keramik auf ca. 1000 A/cm² im Nullfeld beschränken und zu einer starken Magnetfeldabhängigkeit der kritischen Stromdichten führen. Diese Nachteile der granularen Struktur werden in epitaktischen Schichten aufgrund des Fehlens von Korngrenzen nicht beobachtet. Man hat daher versucht, dünne einkristalline Schichten durch physikalische Verfahren, z. B. durch Sputtern oder Laser-Ablation herzustellen. Gegenüber diesen Verfahren besitzt das Verfahren der Metal Organic Chemical Vapor Deposition (MOCVD) eine Reihe von Vorteilen: Die Abscheidung erfolgt bei einem Unterdruck von etwa 10 mbar und erfordert daher keine teure Hochvakuumanlage. Außerdem kommt es beim Beschichten zu einer großen Streuung, so daß sowohl geometrisch kompliziert aufgebaute Substrate an ihrer gesamten Oberfläche als auch große Flächen gleichmäßig beschichtet werden können.

Nachteilig an dieser Methode ist die thermische Empfindlichkeit der verwendeten Ausgangsverbindungen, insbesondere derer enthaltend die Erdalkalimetalle Ca, Sr und Ba. Diese Verbindungen müssen bei 10 mbar bei Temperaturen bis 250 °C flüchtig sein. Beispielsweise ist aus der DE-OS 3 827 069 bekannt, daß man zur Herstellung von $YBa_2Cu_3O_x$ unter anderem den Barium-Chelatkomplex des 2,2,6,6-Tetramethylheptandions einsetzt.

Es hat sich aber gezeigt, daß sich die bekannten Erdalkali-Chelatkomplexe des 2,2,6,6-Tetramethylheptandions bereits während der Verdampfung merklich zersetzen und ihre Verdampfungstemperatur von Charge zu Charge in nicht reproduzierbarer Weise schwankt. Dies macht eine exakte Prozeßführung in der Praxis schwierig und erhöht den Ausschuß. Auf diese Schwierigkeiten wird z. B. hingewiesen in Superconductor Week, Sept. 10, 1990, p. 2 - 3.

Die Synthese von Chelatverbindungen der seltenen Erden, die sich von 2,2,6,6-Tetramethylheptandion ableiten, ist im Prinzip bekannt und wird durch K. J. Eisentraut et al., J. Am. Chem. Soc. 87 (1965) 5254 beschrieben. Dabei werden ein Salz der seltenen Erden, das erwähnte Heptandion und eine Base in Ethanol/Wasser gelöst, die Lösungen vereinigt und durch Zugabe von Wasser der Niederschlag an Chelat ausgefällt. Die Chelate der seltenen Erden kristallieren kristallwasserfrei. Das Verfahren kann auch auf die Herstellung analoger Chelatverbindungen der Erdalkali-Metalle übertragen werden.

G. S. Hammond et al., Inorg. Chem. 2 (1963) 73 geben eine Methode zur Herstellung von Ba-Chelaten des 2,2,6,6-Tetramethylheptandions an, bei dem letzteres mit einer wäßrigen Lösung von $Ba(OH)_2$ so lange geschüttelt wird bis die Diketon-Phase verschwunden und das Ba-Chelat ausgefallen ist.

Genauere Untersuchungen haben ergeben, daß die Reaktionsprodukte bei diesen Verfahren als Verunreinigungen noch Kristallwasser enthalten, anhaftendes Wasser (Feuchtigkeit), nicht umgesetzte freie Diketon-Liganden sowie Ausgangssalze, z. B. Bariumhydroxid und/oder Bariumnitrat. Durch ein Umkristallisieren werden der freie Ligand und anhaftende Salze entfernt; durch Trocknen im Vakuum läßt sich die Feuchtigkeit entziehen, nicht aber der Kristallwassergehalt. Beim Verdampfen im CVD-Prozeß schmelzen die Substanzen im eigenen Kristallwasser, wobei es auch zur Hydrolyse kommt. Daher sind Verdampferrückstände von 20 bis 30 % der eingewogenen Menge, im wesentlichen aus Bariumoxid oder Bariumhydroxid bestehend, keine Seltenheit.

Aufgabe der Erfindung war es daher, neue Erdalkali-Verbindungen aufzufinden, die konstante Verdampfungsraten zeigen und insbesondere leichter flüchtig sind, die sich deshalb besser kontrollierbar abscheiden lassen.

Gelöst wird diese Aufgabe durch Erdalkalisalze des 2,2,6,6-Heptamethylheptandions der allgemeinen Formel I

$$M^{2+}(C_{11}H_{19}O_2)^-_2 \cdot L_k \qquad (I)$$

wobei M für Calcium, Strontium und insbesondere Barium, L für einen Liganden und k für eine Zahl von 1 bis 3 steht, die dadurch gekennzeichnet sind, daß sie frei sind von Kristallwasser und sie als Liganden L einen aliphatischen Ether mit mindestens zwei Sauerstoffatomen im Molekül enthalten. Vorzugsweise ist der aliphatische Ether ein Bis-methylether oder Bis-ethylether, insbesondere des Ethylenglykols oder Diethylenglykols. Ether des Tri- und Tetraethylenglycols sind ebenfalls geeignet.

Die erfindungsgemäßen Erdalkali-Chelatkomplexe lassen sich dadurch herstellen, daß man einen kristallwasserhaltigen Erdalkali-Chelatkomplex des 2,2,6,6-Tetramethylheptandions aus einem aliphatischen Ether umkristallisiert, der mindestens zwei Sauerstoffatome im Molekül enthält. Beim Umkristallisieren wird das bisherige Kristallwasser vollständig aus der Koordinationssphäre verdrängt und durch den Ether-Liganden ersetzt. Der Ethergehalt variiert meist zwischen 1,5 und 2,3 Molekülen pro Formeleinheit und hängt im wesentlichen von der Konzentration beim Umkristallisieren ab. Je konzentrierter die Lösung ist, umso weniger Ether ist in der Verbindung.

Da die neuen Verbindungen kein Wasser enthalten, kann während ihrer Verdampfung keine Hydrolyse stattfinden. Der Verdampfungsprozeß beim MOCVD-Verfahren verläuft daher über mehrere Stunden stabil. Unter trockenen Bedingungen sind die neuen Verbindungen unbegrenzt lagerstabil. Im Gegensatz zu Wasser handelt es sich bei den erfindungsgemäß eingesetzten Ethern um mehrzähnige Liganden. Dadurch wird die Koordinationssphäre des Erdalkalimetalls leichter abgesättigt, was beim CVD-Prozeß zu niedrigeren Verdampfungstemperaturen, guten Ausbeuten und guter Reproduzierbarkeit führt.

Abbildung 1 zeigt die Arrhenius-Plots von Ba-(thd)$_2$-Solvaten in Wasser, 1,2-Dimethoxyethan (DME) und Diethylenglycoldimethylether (Dgl) und die aus den Geradensteigungen errechneten Aktivierungsenergien für die Verdampfung. Daraus erkennt man, daß das Hydrat und das DME-Etherat zwar ungefähr gleiche Aktivierungsenergien besitzen, das Etherat zeigt aber um ca. 50 % höhere Verdampfungsraten.

Das Dgl-Etherat zeigt zwar gleiche Verdampfungsraten wie das Hydrat, es besitzt aber eine geringere Aktivierungsenergie. Dadurch werden die Ergebnisse bei der Beschichtung besser reproduzierbar, da sich Schwankungen in der Verdampfungstemperatur weniger stark in Ratenschwankungen äußern.

Die erfindungsgemäßen Erdalkali-Chelatkomplexe eignen sich daher sehr gut für den MOCVD-Prozeß. Ganz besonders eignen sie sich für ein Verfahren zur Beschichtung eines inerten Substrats mit einem Hochtemperatur-Supraleiter, der Barium, Calcium oder Strontium enthält. Bei diesem Verfahren werden flüchtige Verbindungen der einzelnen den Hochtemperatur-Supraleiter aufbauenden Metalle in getrennten Verdampfern auf unterschiedliche Temperatur aufgeheizt, der entstehende Dampf mit einem inerten Trägergas mit einem Sauerstoffstrom vereinigt und das Gasgemisch an einer ca. 850 °C heißen Substratoberfläche pyrolisiert, wobei sich die Metalloxide auf dem Substrat niederschlagen. Das beschichtete Substrat wird anschließend langsam, vorzugsweise in ca. einer Stunde, in einer Sauerstoff enthaltenden Atmosphäre auf Zimmertemperatur abgekühlt. Nach einer anderen Ausführungsform der Erfindung wird das Gasgemisch bei ca. 850 °C pyrolysiert und die heißen Pyrolysegase auf einem gekühlten Substrat niedergeschlagen. Das beschichtete Substrat wird anschließend über einen Zeitraum von wenigstens einer Stunde in einer Sauerstoff enthaltenden Atmosphäre erhitzt. Damit soll der Sauerstoffgehalt der Beschichtung so weit erhöht werden, daß ein Supraleiter vorliegt. Dieses Verfahren ist dadurch gekennzeichnet, daß als flüchtige Erdalkali-Verbindung ein erfindungsgemäßer Erdalkali-Chelatkomplex eingesetzt wird. Eine bevorzugte Vorrichtung zur Herstellung dünner Schichten aus Metallmischoxiden aus organischen Metallverbindungen auf einem Substrat nach dem CVD-Verfahren ist bekannt aus der deutschen Patentanmeldung DE-OS 40 06 489.1.

Die erfindungsgemäß abgeschiedenen, Erdalkali enthaltenden Supraleiter, z. B. Bi$_2$Sr$_2$CaCu$_2$O$_8$ oder Bi$_2$Sr$_2$Ca$_2$Cu$_3$O$_{20}$ werden in O$_2$ enthaltender Atmosphäre abgekühlt. Bei Verbindungen des Types YBa$_2$Cu$_3$O$_7$ muß längere Zeit in einer Sauerstoff enthaltenden Atmosphäre erhitzt werden, um die Umwandlung in die orthorhombische Phase zu vervollständigen. Hierfür eignet sich z. B. eine Haltestufe von 10 bis 30 Minuten bei 400 - 500 °C.

Die Erfindung wird durch die Beispiele näher erläutert. Dabei bedeutet „"thd" das 2,2,6,6-Tetramethylheptadion-Anion und "DME" 1,2-Dimethoxyethan.

Beispiel 1

25,55 g Ba(thd)$_2$•0,8 H$_2$O werden bei Zimmertemperatur in 175 ml 1,2-Dimethoxyethan gelöst (= 146 g/l). Im Kühlschrank bei 8 °C fallen farblose quaderförmige Kristalle aus. Die überstehende Lösung wird in der Kälte entfernt (z. B. durch Abpipettieren) und die Kristalle im Vakuum von anhaftender Flüssigkeit befreit.
Ausbeute: 18,3 g Ba(thd)$_2$•1,60 DME.

Beispiel 2

Versuchsdurchführung wie Beispiel 1.
20 g Ba(thd)$_2$•0,75 H$_2$O, 300 ml 1,2-Dimethoxyethan (= 67 g/l), Fällung im Eisfach bei - 18°C.
Ausbeute: 14,8 g Ba(thd)$_2$•2,27 DME.

Beispiel 3

10 g Ba(thd)$_2$•0,3 H$_2$O werden bei 60 °C in 20 ml Diethylenglycoldimethylether gelöst (= 500 g/l). Im Kühlschrank bei 8 °C fallen farblose quaderförmige Kristalle aus. Die überstehende Lösung wird in der Kälte entfernt (z. B. durch Abpipettieren) und die Kristalle im Vakuum von anhaftender Flüssigkeit befreit. Ausbeute: 10,2 g Ba(thd)$_2 \cdot$ 1,52 Dgl (Dgl = Diethylenglycoldimethylether).

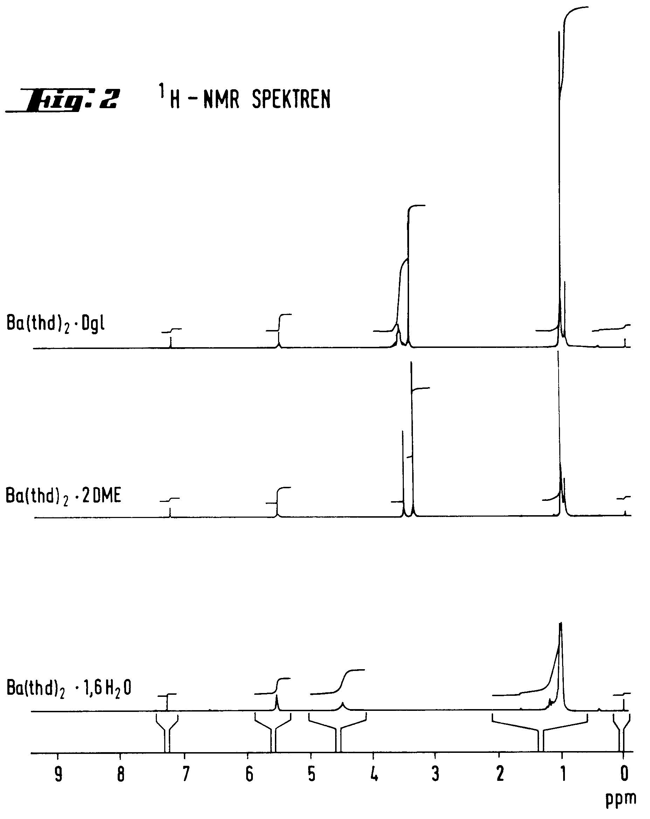

Die Kernresonanzspektren ($^1$H-NMR-Spektren bei 100 MHz, Lösungsmittel CDCl$_3$/TMS) der Verbindungen sind Figur 2, die Verdampfungseigenschaften der Figur 1 zu entnehmen. Dabei bedeutet R die anhand des Gewichtsverlustes gemessene Verdampfungsgeschwindigkeit verschiedener Bachelate [mg/min.]. In Figur 1 ist ferner die aus den Geraden entnommene Aktivierungsenergie für die Verdampfung der Verbindungen angegeben, die sich aus der Arrheniusgleichung berechnet.

## Patentansprüche

1. Erdalkali-Chelatkomplex des 2,2,6,6-Tetramethylheptandions mit der allgemeinen Formel (I)

$$M^{2^+}(C_{11}H_{19}O_2)^-_2 \cdot L_k \qquad (I)$$

wobei M für Calcium, Strontium oder Barium, L für einen Liganden und k für eine Zahl von 1 bis 3 steht, dadurch gekennzeichnet, daß er frei ist von Kristallwasser und der Ligand L ein aliphatischer Ether mit mindestens zwei Sauerstoffatomen im Molekül ist.

2. Chelatkomplex gemäß Anspruch 1, dadurch gekennzeichnet, daß der aliphatische Ether ein Bis-Methylether oder ein Bis-Ethylether ist.

3. Chelatkomplex gemäß Anspruch 2, dadurch gekennzeichnet, daß der aliphatische Ether ein Bis-ether von Ethylenglykol oder Diethylenglykol ist.

4. Verfahren zur Herstellung des Erdalkali-Chelatkomplexes gemäß Anspruch 1, dadurch gekennzeichnet, daß man einen kristallwasserhaltigen Erdalkali-Chelatkomplex des 2,2,6,6-Tetramethylheptandions aus einem aliphatischen Ether umkristallisiert, der mindestens zwei Sauerstoffatome im Molekül enthält.

5. Verfahren zur Beschichtung eines inerten Substrats mit einem Hochtemperatur-Supraleiter, der Barium enthält, nach dem CVD-Verfahren, wobei man flüchtige Verbindungen der einzelnen den Hochtemperatursupraleiter aufbauenden Metalle in getrennten Verdampfern auf unterschiedliche Temperatur aufheizt und den entstehenden Dampf mit einem inerten Trägergas mit einem Sauerstoffstrom vereinigt, das Gasgemisch bei ca. 850 °C pyrolysiert, die heißen Pyrolysegase auf einem gekühlten Substrat niederschlägt und das beschichtete Substrat gegebenenfalls anschließend längere Zeit in einer Sauerstoff enthaltenden Atmosphäre erhitzt, dadurch gekennzeichnet, daß als flüchtige Verbindung ein Chelatkomplex gemäß Anspruch 1 verwendet wird.

6. Verfahren zur Beschichtung eines inerten Substrats mit einem Hochtemperatur-Supraleiter, der Barium enthält, nach dem CVD-Verfahren, wobei man flüchtige Verbindungen der einzelnen den Hochtemperatursupraleiter aufbauenden Metalle in getrennten Verdampfern auf unterschiedliche Temperatur aufheizt und den entstehenden Dampf mit einem inerten Trägergas mit einem Sauerstoffstrom vereinigt, das Gasgemisch an einer ca. 850 °C heißen Substratoberfläche pyrolysiert, wobei sich die Metalloxide auf dem Substrat niederschlagen und das beschichtete Substrat gegebenenfalls anschließend längere Zeit in einer Sauerstoff enthaltenden Atmosphäre erhitzt, dadurch gekennzeichnet, daß als flüchtige Verbindung ein Chelatkomplex gemäß Anspruch 1 verwendet wird.

## Patentansprüche für folgende Vertragsstaaten : ES, GR

1. Verfahren zur Herstellung des Erdalkali-Chelatkomplexes des 2,2,6,6-Tetramethylheptadions mit der allgemeinen Formel (I)

$$M^{2^+}(C_{11}H_{19}O_2)^-_2 \cdot L_k \qquad (I)$$

wobei M für Calcium, Strontium oder Barium, L für einen Liganden und k für eine Zahl von 1 bis 3 steht, der frei ist von Kristallwasser und bei dem der Ligand L ein aliphatischer Ether mit mindestens zwei Sauerstoffatomen im Molekül ist, dadurch gekennzeichnet, daß man einen kristallwasserhaltigen Erdalkali-Chelatkomplex des 2,2,6,6-Tetramethylheptandions aus einem aliphatischen Ether umkristallisiert, der mindestens zwei Sauerstoffatome im Molekül enthält.

2. Verfahren gemäß Anspruch 1, dadurch gekennzeichnet, daß der aliphatische Ether ein Bis-Methylether oder ein Bis-Ethylether ist.

3. Verfahren gemäß Anspruch 2, dadurch gekennzeichnet, daß der aliphatische Ether ein Bis-ether von Ethylenglykol oder Diethylenglykol ist.

4. Verfahren zur Beschichtung eines inerten Substrats mit einem Hochtemperatur-Supraleiter, der Barium enthält, nach dem CVD-Verfahren, wobei man flüchtige Verbindungen der einzelnen den Hochtemperatursupraleiter aufbauenden Metalle in getrennten Verdampfern auf unterschiedliche Temperatur aufheizt und den entstehenden Dampf mit einem inerten Trägergas mit einem Sauerstoffstrom vereinigt, das Gasgemisch bei ca. 850 °C pyrolysiert, die heißen Pyrolysegase auf einem gekühlten Substrat niederschlägt und das beschichtete Substrat gegebenenfalls anschließend längere Zeit in einer Sauerstoff enthaltenden Atmosphäre erhitzt, dadurch gekennzeichnet, daß als flüchtige Verbindung ein Chelatkomplex des 2,2,6,6-Tetramethylheptadions mit der allgemeinen Formel (I)

$$M^{2+}(C_{11}H_{19}O)^-_2 \cdot L_k \qquad (I)$$

wobei M für Calcium, Strontium oder Barium, L für einen Liganden und k für eine Zahl von 1 bis 3 steht, der frei ist von Kristallwasser und bei dem der Ligand L ein aliphatischer Ether mit mindestens zwei Sauerstoffatomen im Molekül ist, verwendet wird.

5. Verfahren zur Beschichtung eines inerten Substrats mit einem Hochtemperatur-Supraleiter, der Barium enthält, nach dem CVD-Verfahren, wobei man flüchtige Verbindungen der einzelnen den Hochtemperatursupraleiter aufbauenden Metalle in getrennten Verdampfern auf unterschiedliche Temperatur aufheizt und den entstehenden Dampf mit einem inerten Trägergas mit einem Sauerstoffstrom vereinigt, das Gasgemisch an einer ca. 850 °C heißen Substratoberfläche pyrolysiert, wobei sich die Metalloxide auf dem Substrat niederschlagen und das beschichtete Substrat gegebenenfalls anschließend längere Zeit in einer Sauerstoff enthaltenden Atmosphäre erhitzt, dadurch gekennzeichnet, daß als flüchtige Verbindung ein Chelatkomplex des 2,2,6,6-Tetramethylheptadions mit der allgemeinen Formel (I)

$$M^{2+}(C_{11}H_{19}O_2)^-_2 \cdot L_k \qquad (I)$$

wobei M für Calcium, Strontium oder Barium, L für einen Liganden und k für eine Zahl von 1 bis 3 steht, der frei ist von Kristallwasser und bei dem der Ligand L ein aliphatischer Ether mit mindestens zwei Sauerstoffatomen im Molekül ist, verwendet wird.

**Fig. 1** ARRHENIUS – PLOT DER VERDAMPFUNGSRATEN VON Ba(thd) – SOLVATEN

EP 0 508 345 A2

Fig. 2  ¹H – NMR SPEKTREN

Ba(thd)$_2$·Dgl

Ba(thd)$_2$·2DME

Ba(thd)$_2$·1,6 H$_2$O